# EUROPEAN PATENT SPECIFICATION

(11) **EP 4 273 540 B1**
(45) Date of publication and mention of the grant of the patent: **06.05.2026**
(21) Application number: 21914584.4
(22) Date of filing: 30.12.2021
(51) Int. Cl.: C07H 15/04, G01N 30/02, G01N 30/50, G01N 30/88, G01N 30/72, G01N 30/34

(54) **METHOD FOR DETECTING CONTENT OF GLYCOSAMINOGLYCAN CARBOXYLATED DERIVATIVE IN SAMPLE, AND APPLICATION THEREOF**
VERFAHREN ZUM NACHWEIS DES GEHALTS AN CARBOXYLIERTEM GLYKOSAMINOGLYKANDERIVAT IN EINER PROBE UND ANWENDUNG DAVON
PROCÉDÉ DE DÉTECTION DE LA TENEUR EN DÉRIVÉ CARBOXYLÉ DE GLYCOSAMINOGLYCANE DANS UN ÉCHANTILLON, ET SON APPLICATION

(30) Priority: 31.12.2020 CN 202011624408
(43) Date of publication of application: 08.11.2023
(73) Proprietor: Shenzhen Hepalink Pharmaceutical Group Co., Ltd., Shenzhen, Guangdong 518057 (CN)
(72) Inventor: WANG, Jingwen, Shenzhen, Guangdong 518057 (CN); REN, Lige, Shenzhen, Guangdong 518057 (CN); LIN, Senmao, Shenzhen, Guangdong 518057 (CN); LI, Li, Shenzhen, Guangdong 518057 (CN)
(74) Representative: Croce, Valeria
(86) International application number: PCT/CN2021/142793
(87) International publication number: WO 2022/143853

(56) References cited:
- WO-A1-2019/149179
- WO-A1-2019/149179
- CN-A- 102 329 397
- CN-A- 105 744 940
- CN-A- 106 188 171
- US-A1- 2017 159 237

## Description

### FIELD OF THE INVENTION

The present application belongs to the technical field of analytical chemistry, and specifically relates to a method for detecting the content of a carboxylated glycosaminoglycan derivative in a sample and use thereof, and especially to a method for detecting the content of a carboxylated glycosaminoglycan derivative in a sample and use thereof with high specificity, high accuracy, good precision, low limit of quantitation and low limit of detection.

### BACKGROUND OF THE INVENTION

The prior art, such as patents CN105744940A and CN111670038A, reported that carboxylated glycosaminoglycan derivatives have anti-tumor and anti-metastasis activity as drugs, which have wide application prospects. The carboxylated glycosaminoglycan derivative is a bicarboxylic acid derivative obtained from unfractionated heparin (UFH) by a two-step oxidation reaction, where the uronic acid vicinal diol structure is oxidized and ring-opened, and the two-step oxidation reaction includes (1) two adjacent alcohol groups on the uronic acid of the glycosaminoglycan are oxidized and the ring is opened to form a dialdehyde structure, and (2) the dialdehyde structure is further oxidized to obtain the dicarboxylic acid structure; the carboxylated glycosaminoglycan derivative belongs to heparin derivatives, which is a linear and structurally inhomogeneous mucopolysaccharide substance. In drug metabolism studies, the drug content of biological samples is required to evaluate the pharmacokinetic properties of the drug. It is difficult to directly detect the intact structure in biological samples; additionally, due to the presence of endogenous substances in biological samples, such as proteins and phospholipids, conventional polysaccharide detection methods are susceptible to the interference of endogenous substances, and thus cannot quantitatively determine the carboxylated glycosaminoglycan derivative. Therefore, it has been an urgent problem to be solved about how to provide an accurate method for detecting a carboxylated glycosaminoglycan derivative.

### SUMMARY OF THE INVENTION

The following is a summary of the subject matter described in detail herein. The summary is not intended to limit the protection scope of the claims.

In view of the deficiencies of the prior art, an object of the present application is to provide a method for detecting the content of a carboxylated glycosaminoglycan derivative in a sample and use thereof, in particular to a method for detecting the content of a carboxylated glycosaminoglycan derivative in a sample and use thereof with high specificity, high accuracy, good precision, low limit of quantitation and low limit of detection.

To achieve the object, the present application adopts the technical solutions described below.

**In** a first aspect, the present application provides a method for detecting the content of a carboxylated glycosaminoglycan derivative in a sample. The method includes the following steps:
(1) hydrolyzing a sample containing a carboxylated glycosaminoglycan derivative to obtain a hydrolysate containing a compound of formula (I): 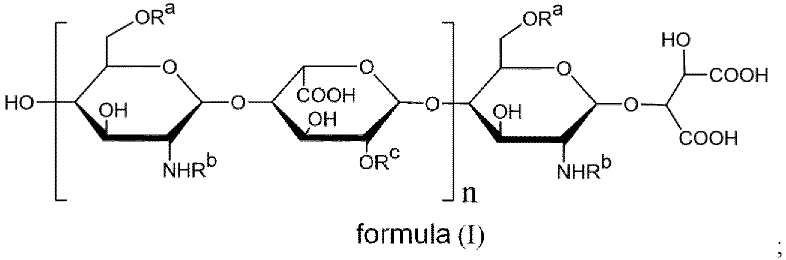 wherein, each R^{a} is independently -SO₃H or -H, each R^{b} is independently H, -SO₃H or -C(O)CH₃, each R^{c} is independently -SO₃H or -H, and n is 0, 1, 2, 3, 4 or 5;
(2) detecting the hydrolysate obtained in step (1) by liquid chromatography tandem mass spectrometry; and
(3) hydrolyzing solutions containing different gradient concentrations of the carboxylated glycosaminoglycan derivative as a standard according to the method of step (1); detecting mass spectral signal peak areas of the compound of formula (I) in the hydrolysates of the solutions containing different concentrations of the standard according to the method of step (2); establishing a standard curve between the contents of the carboxylated glycosaminoglycan derivative standard and the mass spectral signal peak areas, and calculating the content of the carboxylated glycosaminoglycan derivative in the sample based on the standard curve and the mass spectral signal peak area of the compound of formula (I) determined according to the method of step (2); the carboxylated glycosaminoglycan derivative is a glycosaminoglycan compound including a structural unit of formula (II) and optionally a structural unit of formula (III): 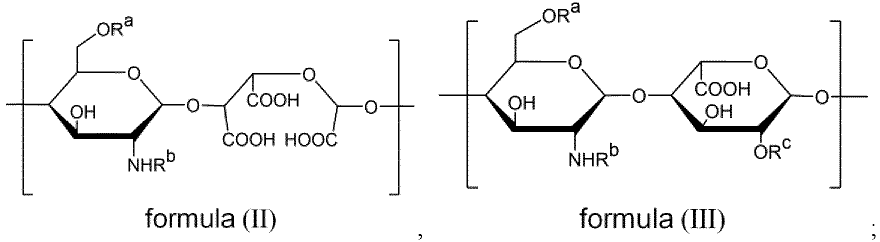 wherein, each R^{a} is independently -SO₃H or -H, R^{b} is independently H, -SO₃H or -C(O)CH₃, and R^{c} is independently -SO₃H or -H.

Preferably, the carboxylated glycosaminoglycan derivative is obtained by a two-step oxidation reaction, including: (1) oxidizing two adjacent alcohol groups on the uronic acid of the glycosaminoglycan and ring-opening to form a dialdehyde structure, and (2) further oxidizing the dialdehyde structure to obtain a dicarboxylic acid structure; and wherein the glycosaminoglycan is heparin or heparan sulfate.

Preferably, the compound of formula (I) is selected from the group consisting of the following structural formulas: 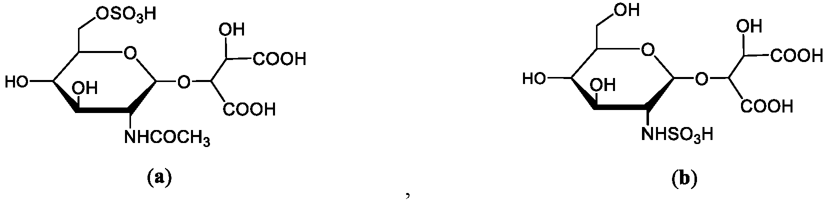 or 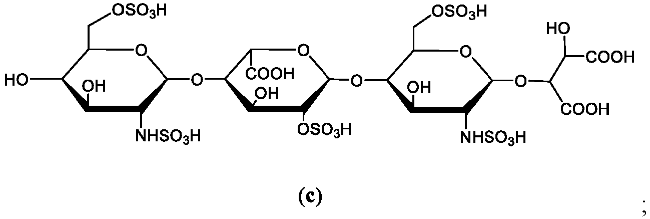
wherein, a mass spectral signal of compound (a) is MS (ESI, neg.ion) m/z: 432.0 [M-H]⁻;
a mass spectral signal of compound (b) is MS (ESI, neg.ion) m/z: 390.0 [M-H]⁻;
a mass spectral signal of compound (c) is MS (ESI, neg.ion) m/z: 522.98 [M-2H]²⁻.

The carboxylated glycosaminoglycan derivative involved in the present application includes the structural unit of formula (II) and optionally the structural unit of formula (III), that is, the hexuronic acid structure in the glycosaminoglycan compound is partially or completely ring-opened.

The carboxylated glycosaminoglycan derivative involved in the present application can be hydrolyzed to obtain the compound of formula (I), a reaction mechanism of which is shown in Schemes 1 and 2, wherein, each R^{a} is independently -SO₃H or -H, each R^{b} is independently H, - SO₃H or -C(O)CH₃, each R^{c} is independently -SO₃H or -H, and n is 0, 1, 2, 3, 4 or 5.

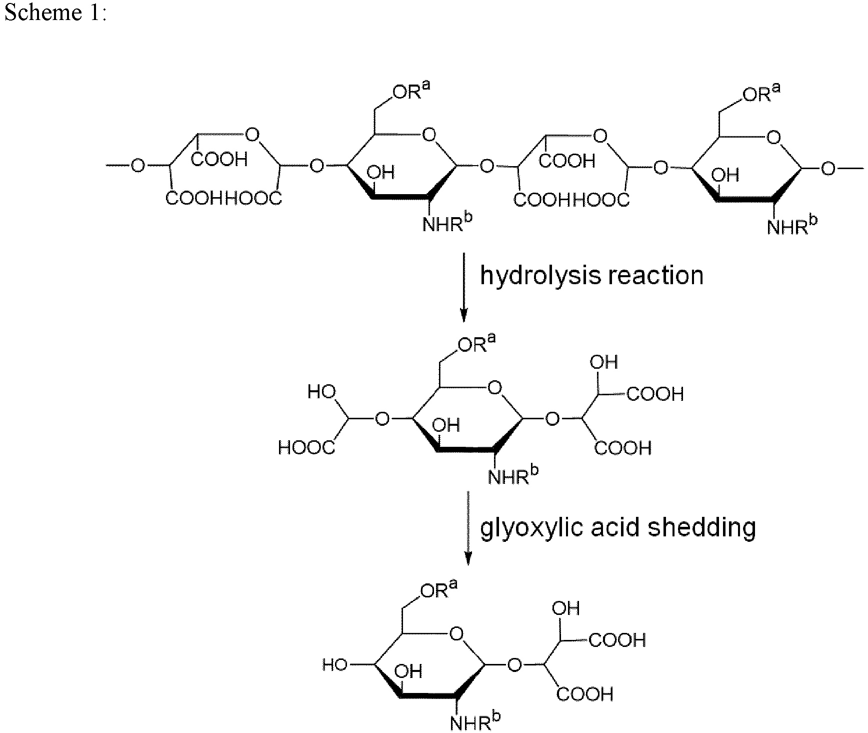

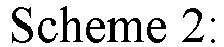

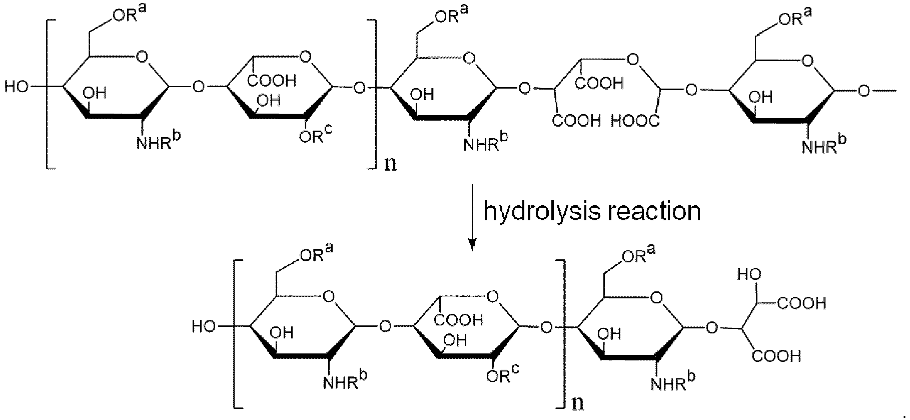

For each polysaccharide chain of the carboxylated glycosaminoglycan derivative involved in the present application, each disaccharide structural unit is arranged in any order.

Preferably, the carboxylated glycosaminoglycan derivative has a weight average molecular weight of 3000-20000 Da, such as 3000 Da, 5000 Da, 7000 Da, 8000 Da, 9000 Da, 10000 Da, 11000 Da, 12000 Da, 13000 Da, 13500 Da, 14000 Da, 16000 Da, 18000 Da or 20000 Da, etc. Other specific point values within this numerical range all can be selected and will not be listed here. The weight average molecular weight is preferably 7000-14000 Da, further preferably 8000-13500 Da.

The carboxylated glycosaminoglycan derivative has a ring-opening degree of 10-100%, such as 10%, 20%, 25%, 30%, 40%, 50%, 60%, 70%, 80%, 90% or 100%, etc. Other specific point values within this numerical range all can be selected and will not be listed here. The ring-opening degree is preferably 25-80%, further preferably 25-60%.

The term "ring-opening degree" in the present application refers to a ratio of the number of ring-opened uronic acid residues to the total number of uronic acid residues, which is detected and calculated with reference to the nuclear magnetic resonance method in the document: Guerrini, M., Guglieri, S, Naggi, A, Sasisekharan, R, (2007). Low molecular weight heparins: Structural differentiation by bidimentional nuclear magnetic resonance spectroscopy. Seminars in Thrombosis and Hemostasis, 33, 478-487.

Preferably, in step (1), a method of hydrolyzing the sample containing a carboxylated glycosaminoglycan derivative is heating.

Preferably, the heating is performed at 70-100°C, such as 70°C, 75°C, 80°C, 82°C, 85°C, 87°C, 95°C, 100°C, etc. Other specific point values within this numerical range all can be selected and will not be listed here. Preferably, the heating is performed at 85-95°C.

Preferably, the heating is performed for 12-168 h, such as 12 h, 16 h, 24 h, 36 h, 48 h, 55 h, 60 h, 70 h, 72 h, 75 h, 78 h, 80 h, 90 h, 96 h, 120 h, 144 h, or 168 h, etc. Other specific point values within this numerical range all can be selected and will not be listed here. Preferably, the heating is performed for 12-120 h.

Under the temperature and time conditions of heating, the carboxylated glycosaminoglycan derivative involved in the present application can produce compounds (a), compounds (b) or compounds (c) with high mass spectral abundance by hydrolysis, and the temperature and time of the reaction are selected with comprehensive consideration of detection efficiency, detection accuracy and precision.

When the sample containing a carboxylated glycosaminoglycan derivative is a biological sample (such as blood or urine), the hydrolysate obtained in step (1) requires detection pretreatment; the pretreatment includes: mixing the hydrolysate with a trifluoroacetic acid solution and an acetonitrile-methanol solution, then performing standing and centrifugation, collecting a supernatant and drying, and then re-dissolving with water.

Preferably, the trifluoroacetic acid solution is added, by volume, in an amount of 0.5-1.5% of the hydrolysate, such as 0.5%, 0.8%, 1.0%, 1.2% or 1.5%, etc. Other specific point values within this numerical range all can be selected and will not be listed here.

Preferably, the trifluoroacetic acid solution has a concentration of 4-6%, such as 4%, 5% or 6%, etc. Other specific point values within this numerical range all can be selected and will not be listed here.

Preferably, the acetonitrile-methanol solution is added, by volume, in an amount of 1-5 times of a volume of the hydrolysate, such as 1 time, 1.5 times, 1.8 times, 2.0 times, 2.2 times, 2.5 times, 3 times, 4 times, 5 times, etc. Other specific point values within this numerical range all can be selected and will not be listed here. The amount is preferably 1-3 times, more preferably 1.5-2.5 times.

Preferably, a volume ratio of acetonitrile to methanol in the acetonitrile-methanol solution is 1:0.5-1:1.5, such as 1:0.5, 1:0.8, 1:1, 1:1.2 or 1:1.5, etc. Other specific point values within this numerical range all can be selected and will not be listed here.

Preferably, the standing is performed at -25 to -15°C, such as -25°C, -20°C, -15°C, etc.; the standing is performed for 15-25 min, such as 15 min, 18 min, 20 min, 22 min, 25 min, etc. Other specific point values within the above numerical ranges all can be selected and will not be listed here.

Preferably, the liquid chromatography is reversed-phase chromatography, size-exclusion chromatography or hydrophilic chromatography.

Preferably, mobile phases of the liquid chromatography are mobile phase A and mobile phase B; the mobile phase A is an aqueous solution of hexafluoroisopropanol and pentylamine; the mobile phase B is an acetonitrile-water solution of hexafluoroisopropanol and pentylamine;
the mobile phase Ais an aqueous solution containing 45-55 mM (such as 45 mM, 48 mM, 50 mM, 52 mM, 55 mM, etc.) hexafluoroisopropanol and 13-17 mM (such as 13 mM, 15 mM, 17 mM, etc.) pentylamine; the mobile phase B is an acetonitrile-water solution containing 45-55 mM (such as 45 mM, 48 mM, 50 mM, 52 mM, 55 mM, etc.) hexafluoroisopropanol and 13-17 mM (such as 13 mM, 15 mM, 17 mM, etc.) pentylamine; the mobile phase B has a volume ratio of acetonitrile to water of 70:30-80:20 (such as 70:30, 75:25, 80:20, etc.). Other specific point values within the above numerical ranges all can be selected and will not be listed here.

Further preferably, the mobile phases of the liquid chromatography are mobile phase A and mobile phase B, which is as below in the table.

| | |
|---|---|
| Mobile phase A | 50 mM hexafluoroisopropanol, 15 mM pentylamine, H₂O |
| Mobile phase B | 50 mM hexafluoroisopropanol, 15 mM pentylamine, acetonitrile/H₂O (75/25, v/v) |

Preferably, the elution process of the liquid chromatography is as below in the table:

| Time (min) | Flow rate (mL/min) | %A | %B | Curve |
|---|---|---|---|---|
| Initial | 0.36 | 98.0 | 2.0 | Initial |
| 3.00 | 0.36 | 98.0 | 2.0 | 6 |
| 10.00 | 0.36 | 80.0 | 20.0 | 6 |
| 15.00 | 0.36 | 60.0 | 40.0 | 6 |
| 15.10 | 0.36 | 10.0 | 90.0 | 6 |
| 18.00 | 0.36 | 10.0 | 90.0 | 6 |
| 18.10 | 0.36 | 98.0 | 2.0 | 6 |
| 22.00 | 0.36 | 98.0 | 2.0 | 6 |

In the present application, the mass spectrometry conditions may exemplarily be the following conditions:

| Conditions | Name/Indicator |
|---|---|
| Mass Spectrometer | Waters Xevo G2-S QTOF |
| Mode | Negative Resolution Mode |
| Set molecular weight | 432.0 Da |
| Capillary voltage | 1.5 kV |
| Sampling cone | 25 V |
| Source compensation voltage | 80 V |
| Source temperature | 120°C |
| Desolvation temperature | 500°C |
| Cone hole gas flow | 50 L/Hr |
| Desolvation gas flow | 800 L/Hr |
| Acquisition start molecular weight | 200 |
| Acquisition termination molecular weight | 2000 |
| Acquisition start time | 1.20 min |
| Acquisition termination time | 6.0 min |

Based on the first aspect, the present application also provides a new compound, and the specific content is as below.

In a second aspect, the present application provides a compound, and the compound has a structure of formula (I): 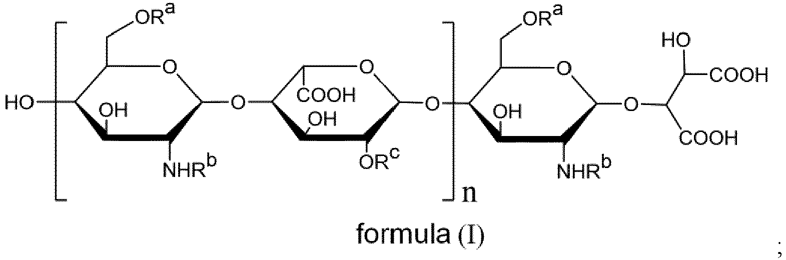 wherein, each R^{a} is independently -SO₃H or -H, each R^{b} is independently H, -SO₃H or -C(O)CH₃, each R^{c} is independently -SO₃H or -H, and n is 0, 1, 2, 3, 4 or 5.

Preferably, the compound has one of the following structures: 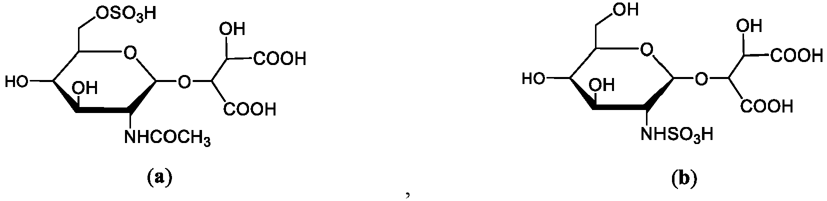 and

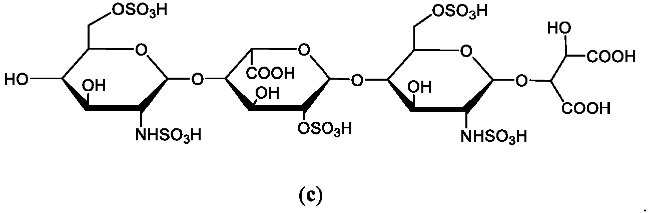

**In** a third aspect, the present application provides use of the method for detecting a carboxylated glycosaminoglycan derivative according to the first aspect in a pharmacokinetic study of a carboxylated glycosaminoglycan derivative.

**In** a fourth aspect, the present application provides use of the method for detecting a carboxylated glycosaminoglycan derivative according to the first aspect in a quality test of a carboxylated glycosaminoglycan derivative pharmaceutical preparation.

Compared with the prior art, the present application has the following beneficial effects.

Because the carboxylated glycosaminoglycan derivative is an inhomogeneous substance, its intact structure is difficult to be detected directly in biological samples. It is found by the inventors of the present application that the carboxylated glycosaminoglycan derivative can be hydrolyzed to reliably produce hydrolysis products compound **(a),** compound **(b)** or compound **(c)** with specific structures as described above, and such compounds can be detected by mass spectrometry. By detecting compounds **(a),** compounds **(b)** or compounds **(c),** a standard curve is established with different concentrations of the carboxylated glycosaminoglycan derivative standard and the corresponding mass spectral peak areas of compounds **(a),** compounds **(b)** or compounds **(c);** then the carboxylated glycosaminoglycan derivative in a sample is hydrolyzed and mass spectral peak areas of compounds **(a),** compounds **(b)** or compounds **(c)** are detected; the sample containing the carboxylated glycosaminoglycan derivative is hydrolyzed and then detecting the mass spectral peak areas of compounds **(a),** compounds **(b)** or compounds **(c)** by liquid chromatography tandem mass spectrometry, and the content of the carboxylated glycosaminoglycan derivative in the sample can be indirectly calculated out based on the standard curve. The detection method has high specificity, high accuracy, good precision, low limit of quantitation and low limit of detection.

After reading and understanding the detailed description, other aspects can be understood.

### BRIEF DESCRIPTION OF THE DRAWING

FIG. 1 is a mass spectrum of compound **(a);**
FIG. 2 is a secondary mass spectrum of compound **(a);**
FIG. 3 is a ¹H-NMR spectrum of compound **(a);**
FIG. 4 is a ¹³C-NMR spectrum of compound **(a);**
FIG. 5 is a ¹³C DEPT 135° NMR spectrum of compound **(a);**
FIG. 6 is a ¹H-¹H COSY spectrum of compound **(a);**
FIG. 7 is a TOCSY spectrum of compound **(a);**
FIG. 8 is an HSQC spectrum of compound **(a);**
FIG. 9 is an HMBC spectrum of compound **(a);**
FIG. 10 is a mass spectrum of compound **(c);**
FIG. 11 is a secondary mass spectrum of compound **(c);**
FIG. 12 is a ¹H-NMR spectrum of compound **(c);**
FIG. 13 is a ¹³C-NMR spectrum of compound **(c);**
FIG. 14 is a ¹³C DEPT 135° NMR spectrum of compound **(c);**
FIG. 15 is a ¹H-¹H COSY spectrum of compound **(c);**
FIG. 16 is a TOCSY spectrum of compound **(c);**
FIG. 17 is a ROESY spectrum of compound **(c);**
FIG. 18 is an HSQC spectrum of compound **(c);** and
FIG. 19 is an HMBC spectrum of compound **(c).**

### DETAILED DESCRIPTION OF THE INVENTION

The technical solutions of the present application are further described below through examples. It should be apparent to those skilled in the art that the examples are only used for a better understanding of the present application and should not be construed as a specific limitation of the present application.

The SD rats involved in the following examples were purchased from Beijing Vital River Laboratory Animal Technology Co., Ltd.

The carboxylated glycosaminoglycan derivative H1011 involved in the following examples was prepared by the preparation method disclosed in Example 3 of patent CN111670038A, which has a weight average molecular weight of 9161 Da and a ring-opening degree of 43.1%.

### Example 1

### Preparation of compound (a), compound (b) and compound (c):

The H1011 (400 mg) was weighed out and dissolved in water (4.0 mL), and the aqueous solution of H1011 was heated to 85°C and reacted for 72 h and then cooled to 25°C; the reaction products were purified and separated by chromatographic technique (chromatographic column: Dionex IonPac AS11-HC, eluent: M and N (see Table 1 for specific components)), and then subjected to desalination and lyophilization to obtain compound (a), compound (b) and compound (c).

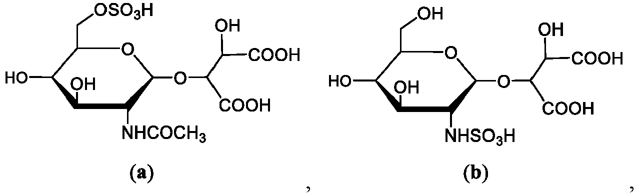

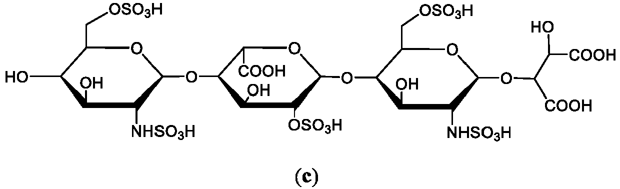

**Table 1**

| Time (min) | Eluent M (%) (2.33 mM sodium dihydrogen phosphate) | Eluate N (%) (2.33 mM sodium dihydrogen phosphate, 1.14 M sodium perchlorate) |
|---|---|---|
| 0 | 97 | 3 |
| 8 | 91 | 9 |
| 20 | 72 | 28 |
| 25 | 0 | 100 |
| 25.1 | 97 | 3 |
| 30 | 97 | 3 |

The compound **(a),** compound **(b)** and **compound** (c) obtained by enrichment were structurally identified by mass spectrometry and nuclear magnetic resonance spectroscopy (1D ¹H-NMR, 1D ¹³C-NMR, ¹³C DEPT 135°, ¹H-¹H COSY, 2D TOCSY, HSQC, HMBC, 2D DOSY).

The characterization result for compound (a) is as follows.

MS (ESI, neg.ion) m/z: 432.0 [M-H]⁻.

¹H-NMR (600 MHz, D₂O): δ 4.87 (d, *J* = 3.5 Hz, 1H), 4.34 (dd, *J* = 11.2, 3.7 Hz, 1H), 4.29 (d, *J* = 4.6 Hz, 1H), 4.27 (dd, *J* = 11.0, 2.1 Hz, 1H), 4.20 (d, *J* = 4.6 Hz, 1H), 4.05 (ddd, *J* = 10.1, 3.7, 2.2 Hz, 1H), 3.96 (dd, *J* = 10.6, 3.5 Hz, 1H), 3.81 (dd, *J* = 10.5, 9.2 Hz, 1H), 3.59 (dd, *J* = 10.1, 9.2 Hz, 1H), 2.07 (s, 3H).

¹³C-NMR (151 MHz, D₂O): δ 24.79, 56.23, 69.39, 71.95, 73.04, 74.14, 76.51, 83.25, 99.56, 177.34, 178.79, 179.99.

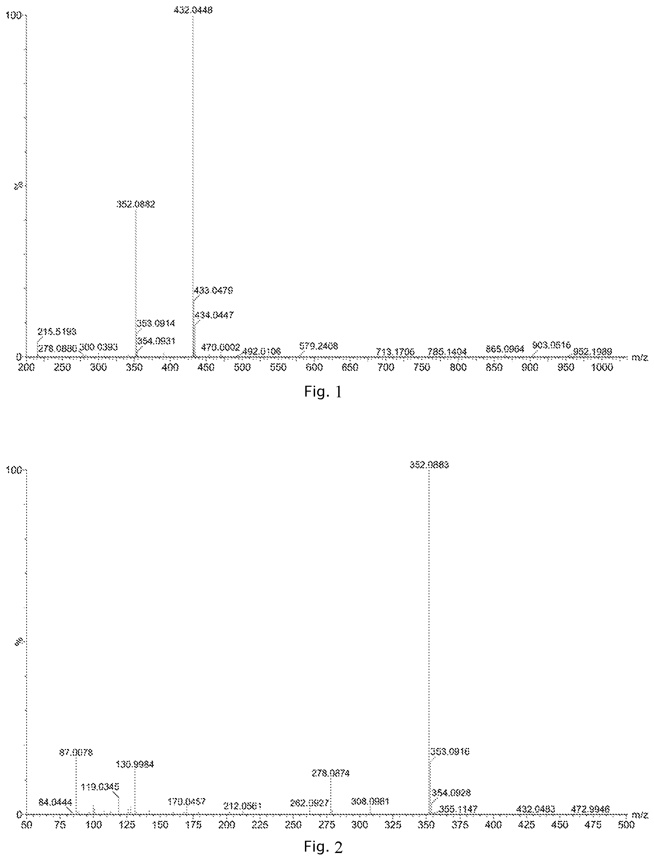

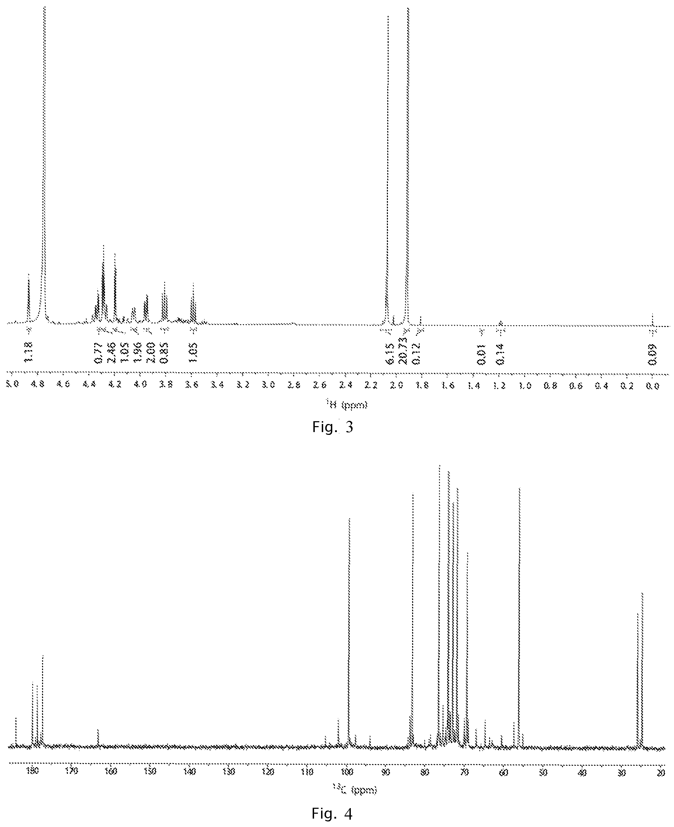

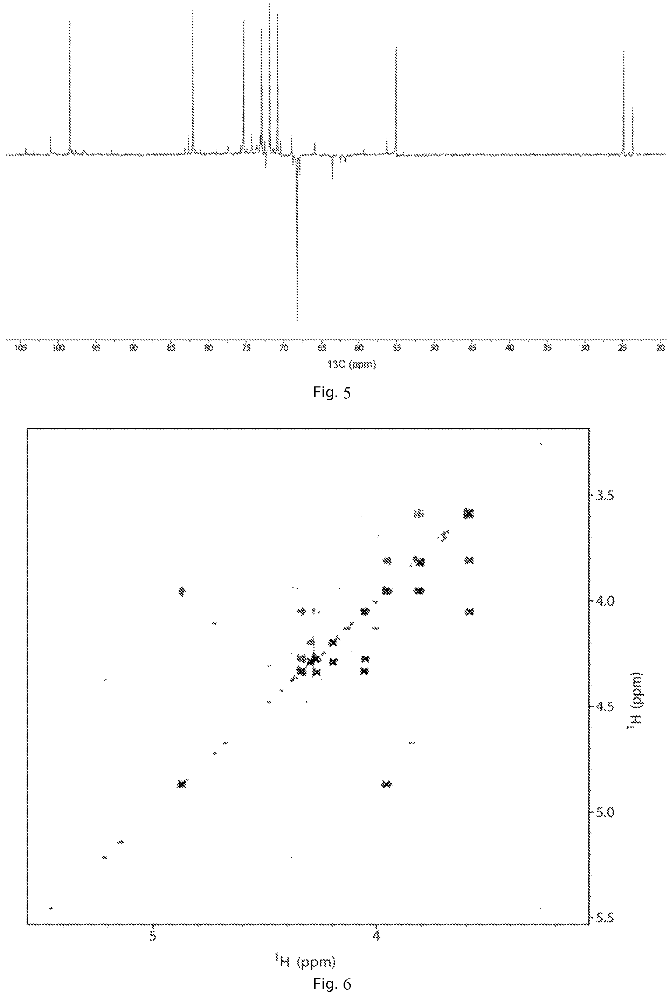

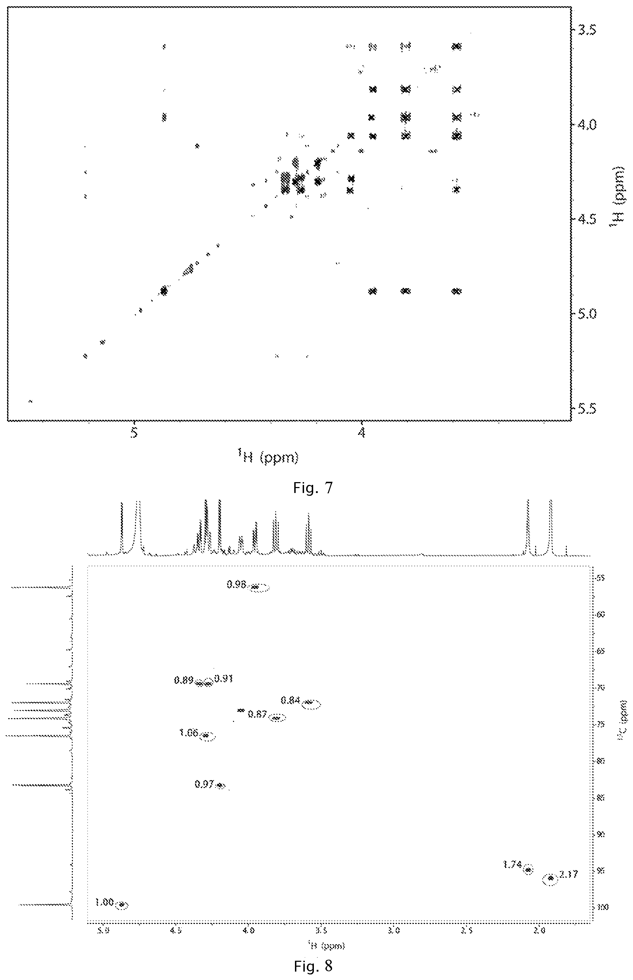

The characterization spectra of compound **(a)** are shown in FIGS. 1-9.

The characterization result for compound **(b)** is as follows.

MS (ESI, neg.ion) m/z: 390.0 [M-H]⁻.

The characterization result for compound **(c)** is as follows.

MS (ESI, neg.ion) m/z: 522.98 [M-2H]²⁻.

¹H-NMR (600 MHz, D₂O): δ 5.37 (d, *J* = 3.5 Hz, 1H), 5.25-5.23 (m, 1H), 5.16 (d, *J* = 3.6 Hz, 1H), 5.15-5.12 (m, 1H), 4.38-4.28 (m, 5H), 4.23 (dd, *J* = 11.4, 2.1 Hz, 1H), 4.19 (dd, *J* = 11.1, 2.1, 1H), 4.13 (dd, *J* = 2.9 Hz, 1H), 3.87 (dt, *J* = 9.7, 2.7 Hz, 1H), 3.84 (dt, *J* = 9.9, 2.6 Hz, 1H), 3.78-3.70 (m, 2H), 3.64 (q, *J* = 7.1 Hz, 1H), 3.60 (dd, *J* = 9.9 Hz, 1H), 3.56 (dd, *J* = 9.5 Hz, 1H), 3.25 (dd, *J* = 10.1, 3.5 Hz, 3H).

¹³C-NMR (151 MHz, D₂O): δ 60.45, 60.63, 68.92, 69.10, 70.44, 70.60, 71.82, 72.44, 72.80, 73.05, 73.50, 74.54, 77.54, 78.74, 79.51, 81.24, 100.90, 101.38, 101.69, 175.69, 176.18, 177.23.

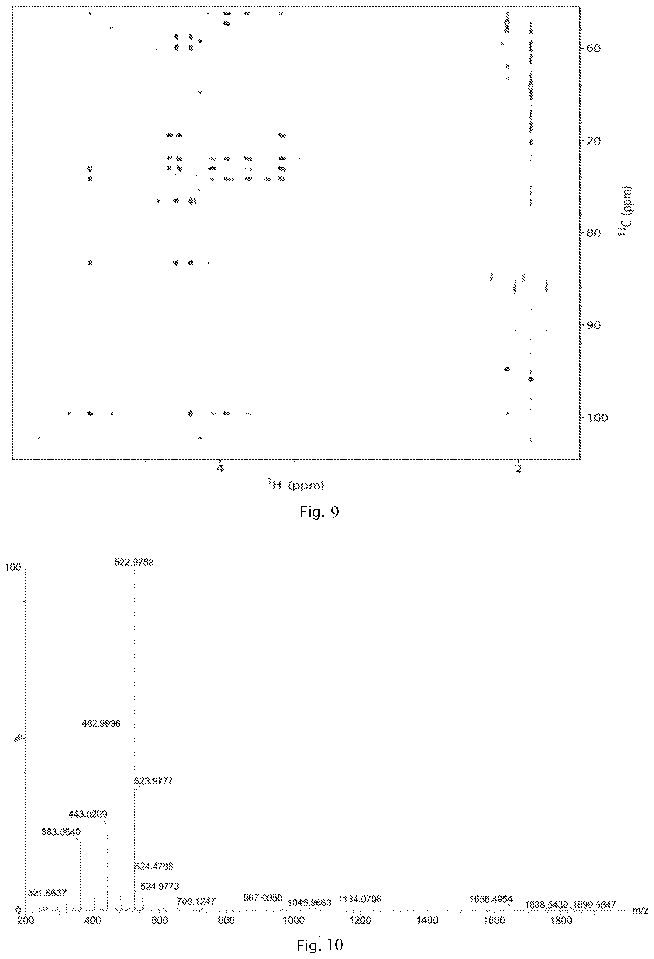

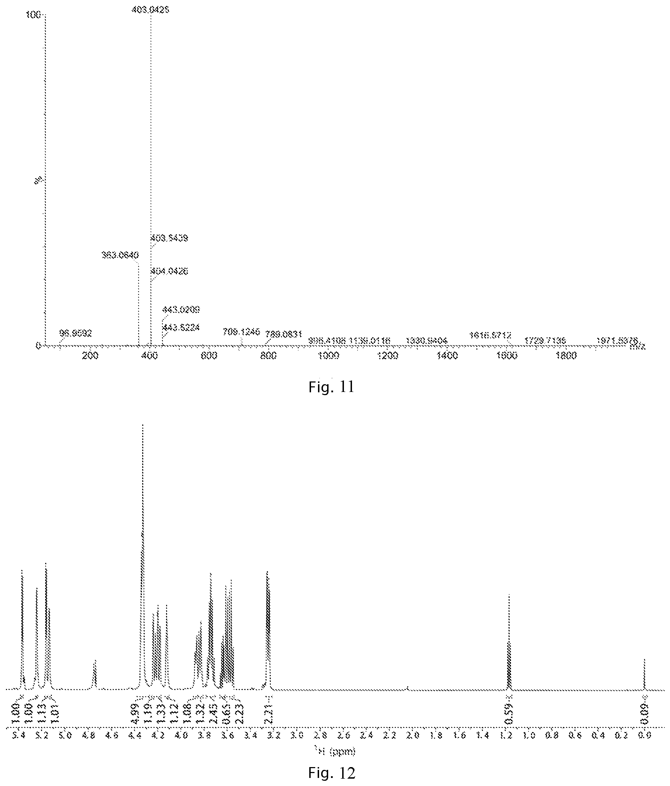

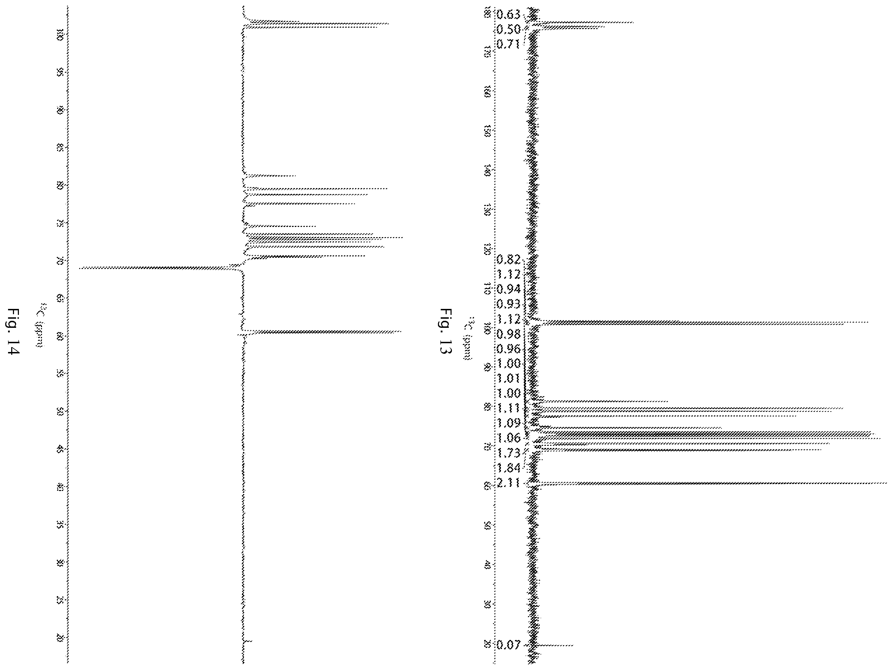

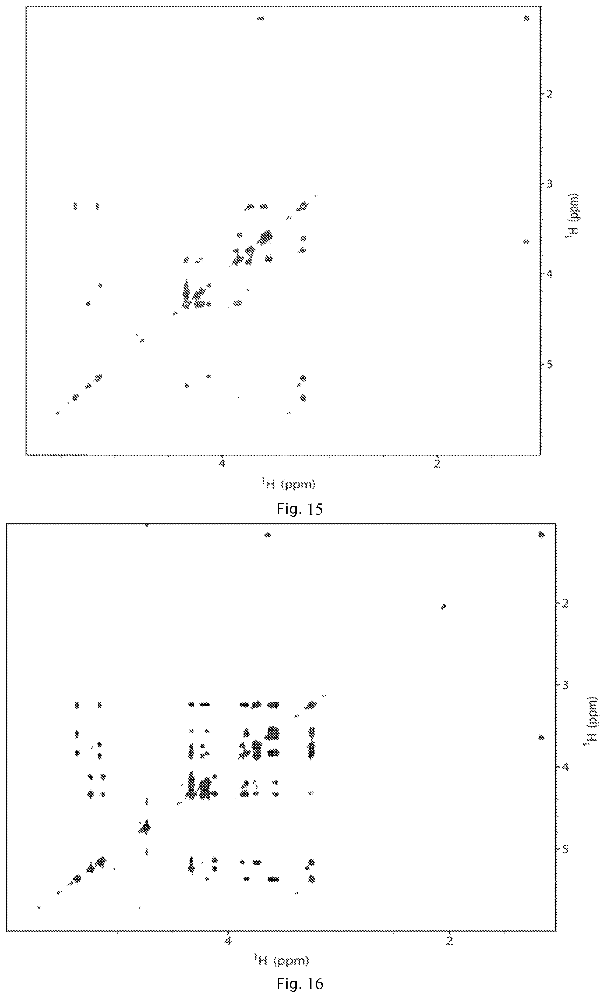

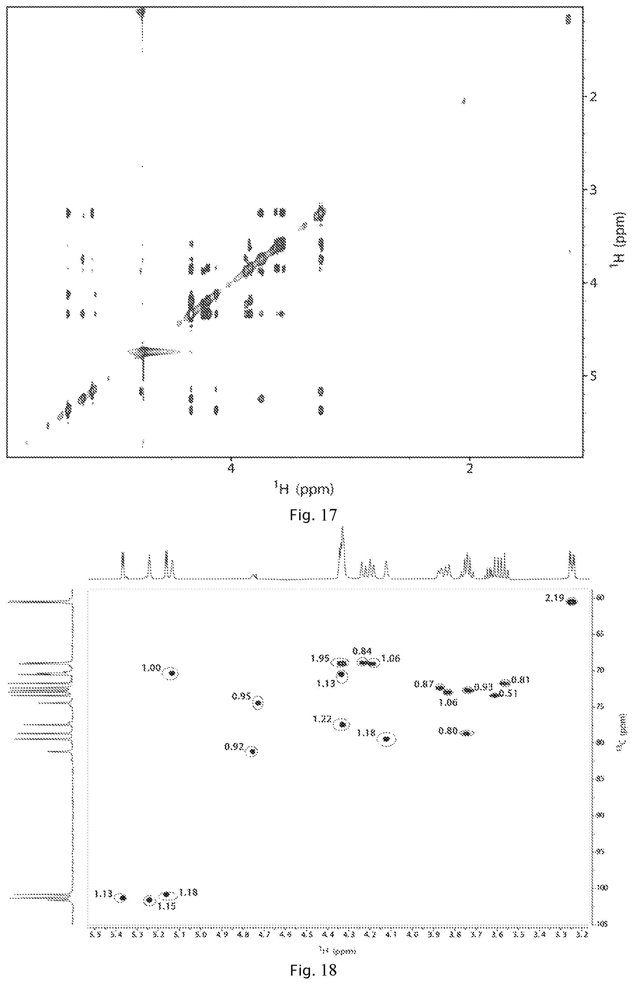

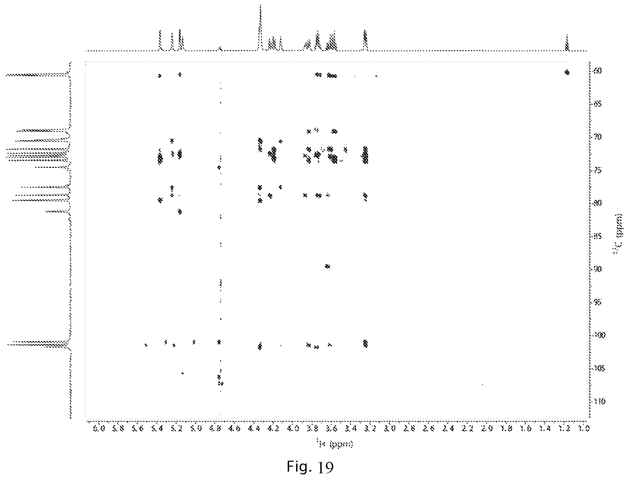

The characterization spectra of compound **(c)** are shown in FIGS. 10-19.

### Example 2

This example applies the method for detecting a carboxylated glycosaminoglycan derivative involved in the present application to a pharmacokinetic study (with compound **(a)** as the detection object), and the specific contents are as follows.

### (1) Test method

(1.1) Experimental animals: 6 healthy adult male SD rats, in which 3 rats were used to collect blank plasma to establish the standard curve and 3 rats were used to perform the plasma drug concentration detection after a single dose in rats.

(1.2) Drug preparation: the carboxylated glycosaminoglycan derivative H1011 was weighed out and prepared into a 60 mg/kg drug solution with water.

(1.3) Administration and sample collection: after administered by subcutaneous injection at a dose of 60 mg/kg, blood was collected at 0, 0.25, 0.5, 1, 2, 4, 6, 8 and 24 h points in time; whole blood was collected, than added in a K2EDTA anticoagulant tube, then centrifuged for 15 min, and separated to obtain a plasma sample.

### (1.4) Standard curve establishment:

(1.4.1) The carboxylated glycosaminoglycan derivative H1011 was weighed out and prepared into a 1 mg/mL aqueous solution, and the H1011 aqueous solution was diluted step by step with the blank plasma as a diluent to prepare 2 µg/mL, 4 µg/mL, 8 µg/mL, 16 µg/mL, 32 µg/mL, 64 µg/mL, 128 µg/mL, and 256 µg/mL standard solutions, and then hydrolyzed at 85°C for 72 h.

(1.4.2) The hydrolyzed standard solution was pre-treated before the detection; the hydrolyzed standard solution was added with one percent volume of trifluoroacetic acid (5%, v/v) and two times volume of acetonitrile/methanol (v/v, 50/50), mixed uniformly, then allowed to stand at - 20°C for 20 min and centrifuged; a supernatant was collected, dried, and then re-dissolved with ultrapure water.

(1.4.3) The liquid chromatography tandem mass spectrometry detection was performed to obtain the spectrum; the chromatography conditions are shown in Table 2 and the mass spectrometry conditions are shown in Table 3.

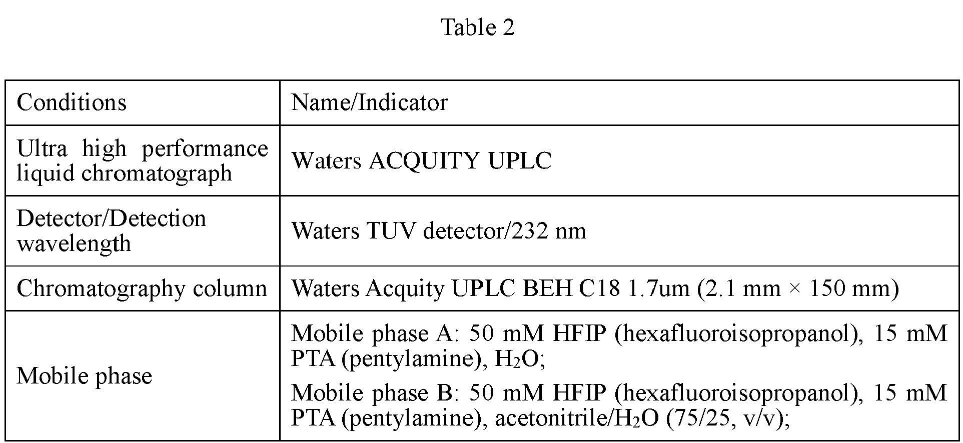

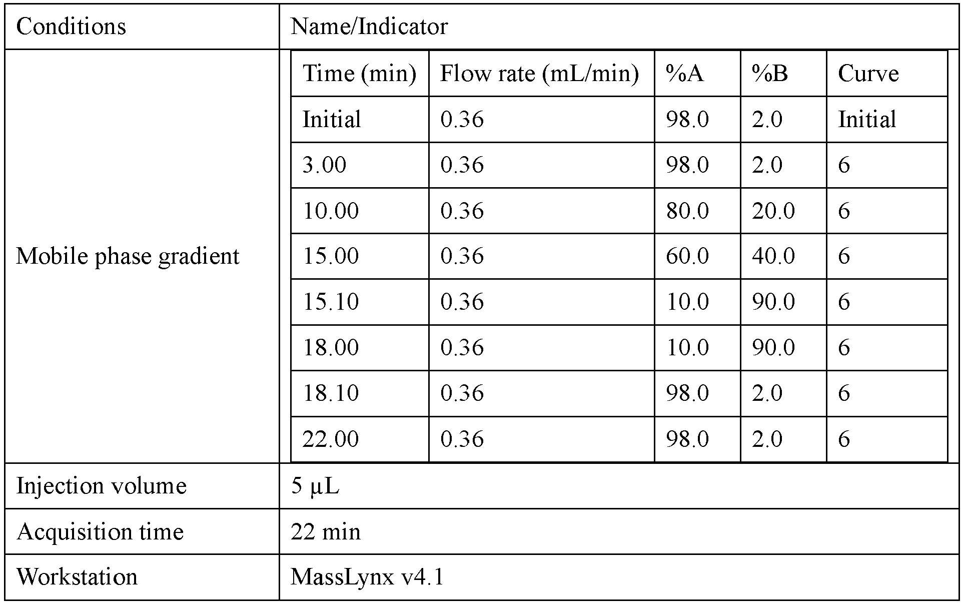

**Table 3**

| Conditions | Name/Indicator |
|---|---|
| Mass Spectrometer | Waters Xevo G2-S QTOF |
| Mode | Negative Resolution Mode |
| Set molecular weight | 432.0 Da |
| Capillary voltage | 1.5 kV |
| Sampling cone | 25 V |
| Source compensation voltage | 80 V |
| Source temperature | 120°C |
| Desolvation temperature | 500°C |
| Cone hole gas flow | 50 L/Hr |
| Desolvation gas flow | 800 L/Hr |
| Acquisition start molecular weight | 200 |
| Acquisition termination molecular weight | 2000 |
| Acquisition start time | 1.20 mins |
| Acquisition termination time | 7.0 mins |

(1.4.4) The standard curve was established between the H1011 standard solution and the mass spectral peak area of compound **(a);** the mass spectral signal of compound **(a)** is MS (ESI, neg.ion) m/z: 432.0 [M-H]⁻; the linear equation is: y = 36.2154x-0.3216; the correlation coefficient is: R² = 0.9987, iwherein y is the mass spectral peak area and x is the concentration of the standard solution.

### (1.5) Detection of the content of H1011 in the plasma sample

(1.5.1) The plasma sample obtained in step (1.3) was hydrolyzed at 85°C for 72 h, then added with one percent volume of trifluoroacetic acid (5%, v/v) and two times volume of acetonitrile/methanol (v/v, 50/50), mixed uniformly, then allowed to stand at -20°C for 20 min and centrifuged; a supernatant was collected, dried, and then re-dissolved with ultrapure water.

(1.5.2) The liquid chromatography tandem mass spectrometry detection was performed to obtain the mass spectral peak area of compound (a); the chromatography conditions are shown in Table 2 and the mass spectrometry conditions are shown in Table 3.

(1.5.3) Based on the mass spectral peak area of compound (a) and the standard curve established in step (1.4), the concentration of H1011 in the plasma sample at each time point was calculated, and pharmacokinetic parameters were calculated based on the drug concentration-time curve.

### (2) The test result is shown in Table 4.

**Table 4**

| Administration route | Dosage | AUC₀₋₂₄ (h*µg/mL) | T_{1/2} (h) | CL (mL/min/kg) |
|---|---|---|---|---|
| i.h. | 60 mg/kg | 537.47 | 3.75 | 0.110 |

### (3) Methodological validation

### (3.1) Specificity

A ultrapure water control solution and a blank plasma control solution were prepared and processed by the same high-temperature hydrolysis and pretreatment procedure as described above, and then subjected to the liquid chromatography tandem mass spectrometry and detected under the same conditions as described above; no signal peak of 432.0 Da, i.e., the mass spectral signal peak of compound **(a),** was observed in the ultrapure water control solution and plasma control solution, indicating that ultrapure water and blank plasma have no interference to the detection, and the detection method has high specificity.

### (3.2) Limit of quantitation and limit of detection

The limit of quantitation of the method was calculated to be 0.8 µg/mL and the limit of detection was 0.2 µg/mL.

### (3.3) Accuracy

The H1011 plasma samples with three concentrations designed as 1 µg/mL, 50 µg/mL and 120 µg/mL were detected; the recovery rate was calculated to be 82.6-110.8%, and the RSD values of 6 experimental results for each concentration were 4.2%, 2.2% and 1.3% in order.

### (3.4) Precision

The 50 µg/mL H1011 plasma solution was selected and detected by two different operators 6 times individually; the RSD of the 6 experimental results of the first operator was 2.0%, the RSD of the 6 experimental results of the second operator was 1.8%, and the RSD of the 12 experimental results of the two operators was 2.1%, all of which meet the acceptable criteria of less than or equal to 10.0%. The method has good precision.

### (3.5) Solution stability

The 50 µg/mL H1011 plasma solution was selected, hydrolyzed and pretreated; the detection result of the sample solution at day 5 is 97.2% of the result at day 0, which meets the criteria.

### Example 3

This example applies the method for detecting a carboxylated glycosaminoglycan derivative involved in the present application to a pharmacokinetic study (with compound (b) as the detection object), and the specific contents are as follows.

### (1) Test method

(1.1) Experimental animals: 6 healthy adult male SD rats, in which 3 rats were used to collect blank plasma to establish the standard curve and 3 rats were used to perform the plasma drug concentration detection after a single dose in rats.

(1.2) Drug preparation: the carboxylated glycosaminoglycan derivative H1011 was weighed out and prepared into a 60 mg/kg drug solution with water.

(1.3) Administration and sample collection: after administered by subcutaneous injection at a dose of 60 mg/kg, blood was collected at 0, 0.25, 0.5, 1, 2, 4, 6, 8 and 24 h points in time; whole blood was collected, than added in a K2EDTA anticoagulant tube, then centrifuged for 15 min, and separated to obtain a plasma sample.

### (1.4) Standard curve establishment

(1.4.1) The carboxylated glycosaminoglycan derivative H1011 was weighed out and prepared into a 1 mg/mL aqueous solution, and the H1011 aqueous solution was diluted step by step with the blank plasma as a diluent to prepare 2 µg/mL, 4 µg/mL, 8 µg/mL, 16 µg/mL, 32 µg/mL, 64 µg/mL, 128 µg/mL and 256 µg/mL standard solutions, and then hydrolyzed at 90°C for 48 h.

(1.4.2) The hydrolyzed standard solution was pre-treated before detection; the hydrolyzed standard solution was added with one percent volume of trifluoroacetic acid (5%, v/v) and two times volume of acetonitrile/methanol (v/v, 50/50), mixed uniformly, then allowed to stand at - 20°C for 20 min and centrifuged; a supernatant was collected, dried, and then re-dissolved with ultrapure water.

(1.4.3) The liquid chromatography tandem mass spectrometry detection was performed to obtain the spectrum; the chromatography conditions are shown in Table 2, the mass spectrometry conditions are shown in Table 3, and the molecular weight is set to be 390.0 Da.

(1.4.4) The standard curve was established between the H1011 standard solution and the mass spectral peak area of compound (**b**); the mass spectral signal of compound **(b)** is MS (ESI, neg.ion) m/z: 390.0 [M-H]⁻; the linear equation is: y = 26.0235x; the correlation coefficient is: R² = 0.9981, in which y is the mass spectral peak area and x is the concentration of the standard solution.

### (1.5) Detection of the content of H1011 in the plasma sample

(1.5.1) The plasma sample obtained in step (1.3) was hydrolyzed at 90°C for 48 h, then added with one percent volume of trifluoroacetic acid (5%, v/v) and two times volume of acetonitrile/methanol (v/v, 50/50), mixed uniformly, then allowed to stand at -20°C for 20 min and centrifuged; a supernatant was collected, dried, and then re-dissolved with ultrapure water.

(1.5.2) The liquid chromatography tandem mass spectrometry detection was performed to obtain the mass spectral peak area of compound **(b);** the chromatography conditions are shown in Table 2, the mass spectrometry conditions are shown in Table 3, and the molecular weight is set to be 390.0 Da.

(1.5.3) Based on the mass spectral peak area of compound **(b)** and the standard curve established in step (1.4), the concentration of H1011 in the plasma sample at each time point was calculated, and pharmacokinetic parameters were calculated based on the drug concentration-time curve.

### (2) The test result is shown in Table 5.

**Table 5**

| Administration route | Dosage | AUC₀₋₂₄ (h*µg/mL) | T_{1/2} (h) | CL (mL/min/kg) |
|---|---|---|---|---|
| i.h. | 60 mg/kg | 483.87 | 3.04 | 0.089 |

### (3) Methodological validation

### (3.1) Specificity

A ultrapure water control solution and a blank plasma control solution were prepared and processed by the same high-temperature hydrolysis and pretreatment procedure as described above, and then subjected to the liquid chromatography tandem mass spectrometry and detected under the same conditions as described above; no signal peak of 390.0 Da, i.e., the mass spectral signal peak of compound **(b),** was observed in the ultrapure water control solution and plasma control solution, indicating that ultrapure water and blank plasma have no interference to the detection, and the detection method has high specificity.

### (3.2) Limit of quantitation and limit of detection

The limit of quantitation of the method was calculated to be 1.1 µg/mL and the limit of detection was 0.55 µg/mL.

### (3.3) Accuracy

The H1011 plasma samples with three concentrations designed as 1 µg/mL, 50 µg/mL and 120 µg/mL were detected; the recovery rate was calculated to be 81.2-115.8%, and the RSD values of 6 experimental results for each concentration were 5.4%, 1.8% and 2.0% in order.

### (3.4) Precision

The 50 µg/mL H1011 plasma solution was selected and detected by two different operators 6 times individually; the RSD of the 6 experimental results of the first operator was 1.6%, the RSD of the 6 experimental results of the second operator was 2.6%, and the RSD of the 12 experimental results of the two operators was 2.2%, all of which meet the acceptable criteria of less than or equal to 10.0%. The method has good precision.

### (3.5) Solution stability

The 50 µg/mL H1011 plasma solution was selected, hydrolyzed and pretreated; the detection result of the sample solution at day 5 is 97.8% of the result at day 0, which meets the criteria.

### Example 4

This example applies the method for detecting a carboxylated glycosaminoglycan derivative involved in the present application to a pharmacokinetic study (with compound **(c)** as the detection object), and the specific contents are as follows.

### (1) Test method

(1.1) Experimental animals: 6 healthy adult male SD rats, in which 3 rats were used to collect blank plasma to establish the standard curve and 3 rats were used to perform the plasma drug concentration detection after a single dose in rats.

(1.2) Drug preparation: the carboxylated glycosaminoglycan derivative H1011 was weighed out and prepared into a 20 mg/kg drug solution with water.

(1.3) Administration and sample collection: after administered by subcutaneous injection at a dose of 60 mg/k, blood was collected at 0, 0.25, 0.5, 1, 2, 4, 6, 8 and 24 h points in time; whole blood was collected, than added in a K2EDTA anticoagulant tube, then centrifuged for 15 min, and separated to obtain a plasma sample.

### (1.4) Standard curve establishment

(1.4.1) The carboxylated glycosaminoglycan derivative H1011 was weighed out and prepared into a 1 mg/mL aqueous solution, and the H1011 aqueous solution was diluted step by step with the blank plasma as a diluent to prepare 2 µg/mL, 4 µg/mL, 8 µg/mL, 16 µg/mL, 32 µg/mL, 64 µg/mL, 128 µg/mL, and 256 µg/mL standard solutions, and then hydrolyzed at 90°C for 36 h.

(1.4.2) The hydrolyzed standard solution was pre-treated before detection; the hydrolyzed standard solution was added with one percent volume of trifluoroacetic acid (5%, v/v) and two times volume of acetonitrile/methanol (v/v, 50/50), mixed uniformly, then allowed to stand at - 20°C for 20 min and centrifuged; a supernatant was collected, dried, and then re-dissolved with ultrapure water.

(1.4.3) The liquid chromatography tandem mass spectrometry detection was performed to obtain the spectrum; the chromatography conditions are shown in Table 2, the mass spectrometry conditions are shown in Table 3, and the molecular weight is set to be 522.98 Da.

(1.4.4) The standard curve was established between the H1011 standard solution and the mass spectral peak area of compound (c); the mass spectral signal of compound (c) is MS (ESI, neg.ion) m/z: 522.98 [M-2H]²⁻; the linear equation is: y = 23.6225x; the correlation coefficient is: R² = 0.9986, in which y is the mass spectral peak area and x is the concentration of the standard solution.

### (1.5) Detection of the content of H1011 in the plasma sample

(1.5.1) The plasma sample obtained in step (1.3) was hydrolyzed at 90°C for 36 h, then added with one percent volume of trifluoroacetic acid (5%, v/v) and two times volume of acetonitrile/methanol (v/v, 50/50), mixed uniformly, then allowed to stand at -20°C for 20 min and centrifuged; a supernatant was collected, dried, and then re-dissolved with ultrapure water.

(1.5.2) The liquid chromatography tandem mass spectrometry detection was performed to obtain the mass spectral peak area of compound (c); the chromatography conditions are shown in Table 2 and the mass spectrometry conditions are shown in Table 3.

(1.5.3) Based on the mass spectral peak area of compound **(c)** and the standard curve established in step (1.4), the concentration of H1011 in the plasma sample at each time point was calculated, and pharmacokinetic parameters were calculated based on the drug concentration-time curve.

### (2) The test result is shown in Table 6.

**Table 6**

| Administration route | Dosage | AUC₀₋₂₄ (h*µg/mL) | T_{1/2} (h) | CL (mL/min/kg) |
|---|---|---|---|---|
| i.h. | 20 mg/kg | 237.44 | 4.04 | 0.073 |

### (3) Methodological validation

### (3.1) Specificity

A ultrapure water control solution and a blank plasma control solution were prepared and processed by the same high-temperature hydrolysis and pretreatment procedure as described above, and then subjected to the liquid chromatography tandem mass spectrometry and detected under the same conditions as described above; no signal peak of 522.98 Da, i.e., the mass spectral signal peak of compound (c), was observed in the ultrapure water control solution and plasma control solution, indicating that ultrapure water and blank plasma have no interference to the detection, and the detection method has high specificity.

### (3.2) Limit of quantitation and limit of detection

The limit of quantitation of the method was calculated to be 2.0 µg/mL and the limit of detection was 1.0 µg/mL.

### (3.3) Accuracy

The H1011 plasma samples with three concentrations designed as 2 µg/mL, 50 µg/mL and 120 µg/mL were detected; the recovery rate was calculated to be 80.3-108.8%, and the RSD values of 6 experimental results for each concentration were 8.9%, 6.5% and 5.4% in order.

### (3.4) Precision

The 50 µg/mL H1011 plasma solution was selected and detected by two different operators 6 times individually; the RSD of the 6 experimental results of the first operator was 6.5%, the RSD of the 6 experimental results of the second operator was 7.2%, and the RSD of the 12 experimental results of the two operators was 7.4%, all of which meet the acceptable criteria of less than or equal to 10.0%. The method has good precision.

### (3.5) Solution stability

The 50 µg/mL H1011 plasma solution was selected, hydrolyzed and pretreated; the detection result of the sample solution at day 5 is 90.2% of the result at day 0, which meets the criteria.

## Claims

1. A method for detecting the content of a carboxylated glycosaminoglycan derivative in a sample, comprising the following steps:
(1) hydrolyzing a sample containing a carboxylated glycosaminoglycan derivative to obtain a hydrolysate containing a compound of formula (I): 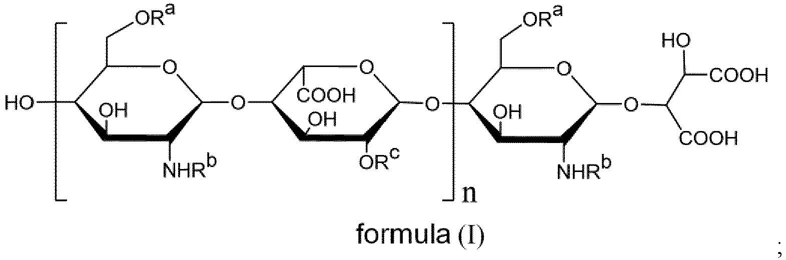 wherein, each R^{a} is independently -SO₃H or -H, each R^{b} is independently H, -SO₃H or -C(O)CH₃, each R^{c} is independently -SO₃H or -H, and n is 0, 1, 2, 3, 4 or 5;
(2) detecting the hydrolysate obtained in step (1) by liquid chromatography tandem mass spectrometry; and
(3) hydrolyzing solutions containing different gradient concentrations of the carboxylated glycosaminoglycan derivative as a standard according to the method of step (1); detecting mass spectral signal peak areas of the compound of formula (I) in the hydrolysates of the solutions containing different concentrations of the standard according to the method of step (2); establishing a standard curve between the contents of the carboxylated glycosaminoglycan derivative standard and the mass spectral signal peak areas; and calculating the content of the carboxylated glycosaminoglycan derivative in the sample based on the standard curve and the mass spectral signal peak area of the compound of formula (I) determined according to the method of step (2);
wherein, the carboxylated glycosaminoglycan derivative is a glycosaminoglycan compound comprising a structural unit of formula (II) and optionally a structural unit of formula (III): 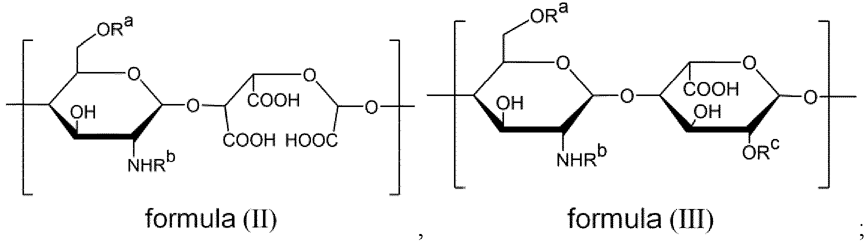
wherein, each R^{a} is independently -SO₃H or -H, R^{b} is independently H, -SO₃H or -C(O)CH₃, and R^{c} is independently -SO₃H or -H.

2. The method of claim 1, wherein
the carboxylated glycosaminoglycan derivative is obtained by a two-step oxidation reaction, comprising: (1) oxidizing two adjacent alcohol groups on the uronic acid of the glycosaminoglycan and ring-opening to form a dialdehyde structure, and (2) further oxidizing the dialdehyde structure to obtain a dicarboxylic acid structure;
wherein the glycosaminoglycan is heparin or heparan sulfate.

3. The method of claim 1, wherein the compound of formula (I) is selected from the group consisting of the following structural formulas: 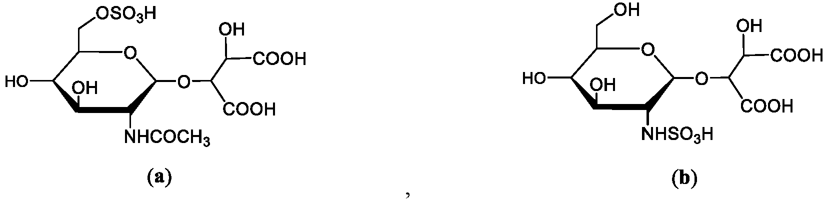 or

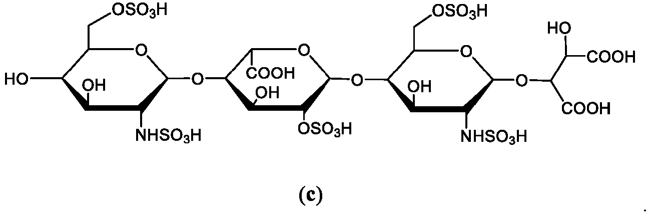

4. The method of claim 1, wherein the carboxylated glycosaminoglycan derivative has a weight average molecular weight of 3000-20000 Da, preferably 7000-14000 Da, and further preferably 8000-13500 Da;
the carboxylated glycosaminoglycan derivative has a ring-opening degree of 10-100%, preferably 25-80%, and further preferably 25-60%.

5. The method of any one of claims 1 to 4, wherein in step (1), a method of hydrolyzing the sample containing a carboxylated glycosaminoglycan derivative is heating;
preferably, the heating is performed at 70-100°C, preferably 85-95°C;
preferably, the heating is performed for 12-168 h, preferably 12-120 h.

6. The method of any one of claims 1 to 5, wherein the liquid chromatography is reversed-phase chromatography, size-exclusion chromatography or hydrophilic chromatography;
preferably, mobile phases of the liquid chromatography are mobile phase A and mobile phase B; the mobile phase A is an aqueous solution of hexafluoroisopropanol and pentylamine; the mobile phase B is an acetonitrile-water solution of hexafluoroisopropanol and pentylamine;
preferably, the mobile phase A is an aqueous solution containing 45-55 mM hexafluoroisopropanol and 13-17 mM pentylamine; the mobile phase B is an acetonitrile-water solution containing 45-55 mM hexafluoroisopropanol and 13-17 mM pentylamine; the mobile phase B has a volume ratio of acetonitrile to water of 70:30-80:20;
preferably, the mobile phases of the liquid chromatography are mobile phase A and mobile phase B, which as below in the table.
| | |
|---|---|
| Mobile phase A | 50 mM hexafluoroisopropanol, 15 mM pentylamine, H₂O |
| Mobile phase B | 50 mM hexafluoroisopropanol, 15 mM pentylamine, acetonitrile/H₂O (75/25, v/v) |

7. The method of any one of claims 1 to 6, wherein, when the sample containing a carboxylated glycosaminoglycan derivative is a biological sample, the hydrolysate obtained in step (1) requires pretreatment before detection; the pretreatment comprises: mixing the hydrolysate with a trifluoroacetic acid solution and an acetonitrile-methanol solution, then performing standing and centrifugation, collecting a supernatant and drying, and then re-dissolving with water;
preferably, the biological sample comprises blood and urine;
preferably, the trifluoroacetic acid solution is added in an amount of 0.5-1.5% of a volume of the hydrolysate;
preferably, the trifluoroacetic acid solution has a concentration of 4-6%;
preferably, the acetonitrile-methanol solution is added in an amount of 1-5 times of a volume of the hydrolysate, preferably 1-3 times, and more preferably 1.5-2.5 times;
preferably, a volume ratio of acetonitrile to methanol in the acetonitrile-methanol solution is 1:0.5-1:1.5;
preferably, the standing is performed at -25 to -15°C for 15-25 min.

8. A compound, which has a structure of formula (I): 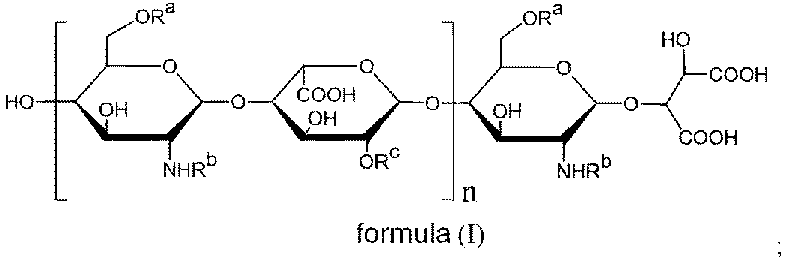 wherein, each R^{a} is independently -SO₃H or -H, each R^{b} is independently H, -SO₃H or -C(O)CH₃, each R^{c} is independently -SO₃H or -H, and n is 0, 1, 2, 3, 4 or 5.

9. The compound of claim 8, wherein the compound has one of the following structures: 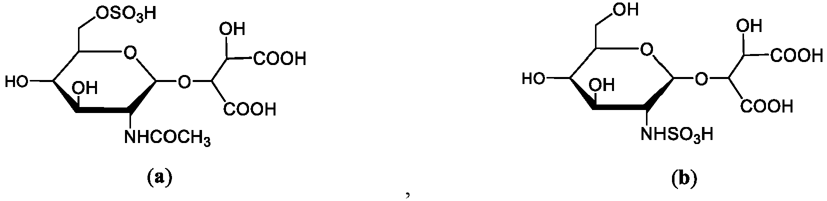 and

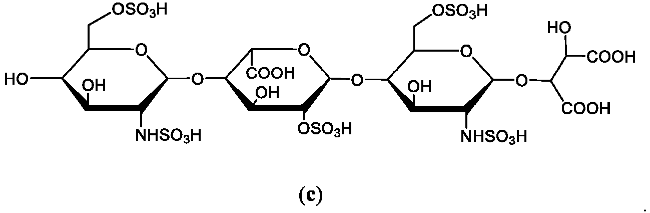

10. Use of the method for detecting a carboxylated glycosaminoglycan derivative of any one of claims 1 to 7 in a pharmacokinetic study of a carboxylated glycosaminoglycan derivative.

11. Use of the method for detecting a carboxylated glycosaminoglycan derivative of any one of claims 1 to 6 in a quality test of a carboxylated glycosaminoglycan derivative pharmaceutical preparation.

## Patentansprüche

1. Verfahren zum Detektieren des Gehalts eines carboxylierten Glycosaminoglycan-Derivats in einer Probe, umfassend die folgenden Schritte:
(1) Hydrolysieren einer Probe, die ein carboxyliertes Glycosaminoglycan-Derivat enthält, zum Erhalten eines Hydrolysats, das eine Verbindung der Formel (I) enthält: 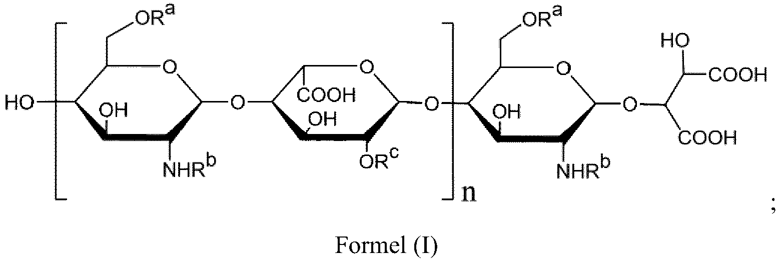 wobei jedes R^{a} unabhängig -SO₃H oder -H ist, jedes R^{b} unabhängig H, -SO₃H oder - C(O)CH₃ ist, jedes R^{c} unabhängig -SO₃H oder -H ist und n 0, 1, 2, 3, 4 oder 5 beträgt;
(2) Detektieren des in Schritt (1) erhaltenen Hydrolysats durch Flüssigkeitschromatographie-Tandem-Massenspektrometrie; und
(3) Hydrolysieren von Lösungen, die verschiedene Gradientenkonzentrationen des carboxylierten Glycosaminoglycan-Derivats als einen Standard enthalten, gemäß dem Verfahren von Schritt (1); Detektieren von massenspektrometrischen Signalpeakflächen der Verbindung der Formel (I) in den Hydrolysaten der Lösungen, die verschiedene Konzentrationen des Standards enthalten, gemäß dem Verfahren von Schritt (2); Erstellen einer Standardkurve zwischen den Gehalten des Standards des carboxylierten Glycosaminoglycan-Derivats und den massenspektrometrischen Signalpeakflächen; und Berechnen des Gehalts des carboxylierten Glycosaminoglycan-Derivats in der Probe bezogen auf die Standardkurve und die massenspektrometrische Signalpeakfläche der Verbindung der Formel (I), die gemäß dem Verfahren von Schritt (2) bestimmt wurde;
wobei das carboxylierte Glycosaminoglycan-Derivat eine Glycosaminoglycan-Verbindung ist, umfassend eine Struktureinheit der Formel (II) und optional eine Struktureinheit der Formel (III): 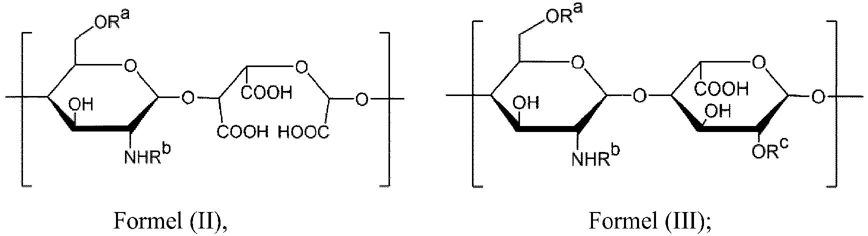
wobei jedes R^{a} unabhängig -SO₃H oder -H ist, R^{b} unabhängig H, -SO₃H oder -C(O)CH₃ ist und R^{c} unabhängig -SO₃H oder -H ist.

2. Verfahren nach Anspruch 1, wobei
das carboxylierte Glycosaminoglycan-Derivat durch eine zweistufige Oxidationsreaktion erhalten wird, umfassend: (1) Oxidieren zweier benachbarter Alkoholgruppen an der Uronsäure des Glycosaminoglycans und Ringöffnen unter Bildung einer Dialdehydstruktur, und (2) weiteres Oxidieren der Dialdehydstruktur zum Erhalten einer Dicarbonsäurestruktur;
wobei das Glycosaminoglycan Heparin oder Heparansulfat ist.

3. Verfahren nach Anspruch 1, wobei die Verbindung der Formel (I) ausgewählt ist aus der Gruppe bestehend aus den folgenden Strukturformeln: 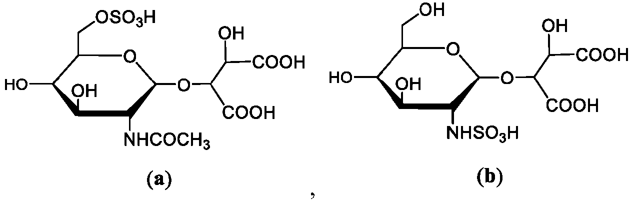 oder

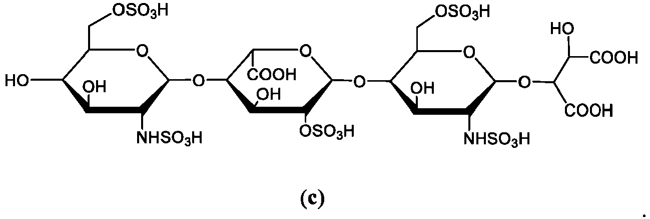

4. Verfahren nach Anspruch 1, wobei das carboxylierte Glycosaminoglycan-Derivat ein gewichtsmittleres Molekulargewicht von 3000 bis 20000 Da, vorzugsweise 7000 bis 14000 Da und weiter bevorzugt 8000 bis 13500 Da aufweist;
wobei das carboxylierte Glycosaminoglycan-Derivat einen Ringöffnungsgrad von 10 bis 100 %, vorzugsweise 25 bis 80 % und weiter bevorzugt 25 bis 60 % aufweist.

5. Verfahren nach einem der Ansprüche 1 bis 4, wobei in Schritt (1) ein Verfahren zum Hydrolysieren der Probe, die ein carboxyliertes Glycosaminoglycan-Derivat enthält, Erhitzen ist;
vorzugsweise das Erhitzen bei 70 bis 100 °C, vorzugsweise 85 bis 95 °C durchgeführt wird;
vorzugsweise das Erhitzen für 12 bis 168 h, vorzugsweise 12 bis 120 h durchgeführt wird.

6. Verfahren nach einem der Ansprüche 1 bis 5, wobei die Flüssigkeitschromatographie Umkehrphasen-Chromatographie, Größenausschluss-Chromatographie oder hydrophile Chromatographie ist;
vorzugsweise mobile Phasen der Flüssigkeitschromatographie mobile Phase A und mobile Phase B sind; wobei die mobile Phase A eine wässrige Lösung von Hexafluorisopropanol und Pentylamin ist; wobei die mobile Phase B eine Acetonitril-Wasser-Lösung von Hexafluorisopropanol und Pentylamin ist;
vorzugsweise die mobile Phase A eine wässrige Lösung ist, die 45 bis 55 mM Hexafluorisopropanol und 13 bis 17 mM Pentylamin enthält; wobei die mobile Phase B eine Acetonitril-Wasser-Lösung ist, die 45 bis 55 mM Hexafluorisopropanol und 13 bis 17 mM Pentylamin enthält; wobei die mobile Phase B ein Volumenverhältnis von Acetonitril zu Wasser von 70:30 bis 80:20 aufweist;
vorzugsweise die mobilen Phasen der Flüssigkeitschromatographie mobile Phase A und mobile Phase B sind, wie unten in der Tabelle angegeben.
| | |
|---|---|
| Mobile Phase A | 50 mM Hexafluorisopropanol, 15 mM Pentylamin, H₂O |
| Mobile Phase B | 50 mM Hexafluorisopropanol, 15 mM Pentylamin, Acetonitril/H₂O (75/25, V/V) |

7. Verfahren nach einem der Ansprüche 1 bis 6, wobei, wenn die Probe, die ein carboxyliertes Glycosaminoglycan-Derivat enthält, eine biologische Probe ist, das in Schritt (1) erhaltene Hydrolysat vor dem Detektieren eine Vorbehandlung erfordert; wobei die Vorbehandlung umfasst: Mischen des Hydrolysats mit einer Trifluoressigsäurelösung und einer Acetonitril-Methanol-Lösung, dann Durchführen eines Stehenlassens und Zentrifugation, Auffangen eines Überstands und Trocknen, und dann Wiederauflösen mit Wasser;
vorzugsweise die biologische Probe Blut und Urin umfasst;
vorzugsweise die Trifluoressigsäurelösung in einer Menge von 0,5 bis 1,5 % eines Volumens des Hydrolysats zugegeben wird;
vorzugsweise die Trifluoressigsäurelösung eine Konzentration von 4 bis 6 % aufweist;
vorzugsweise die Acetonitril-Methanol-Lösung in einer Menge des 1 bis 5-fachen eines Volumens des Hydrolysats, vorzugsweise des 1 bis 3-fachen und noch weiter bevorzugt des 1,5 bis 2,5-fachen zugegeben wird;
vorzugsweise ein Volumenverhältnis von Acetonitril zu Methanol in der Acetonitril-Methanol-Lösung 1:0,5 bis 1:1,5 beträgt;
vorzugsweise das Stehenlassen bei -25 bis -15 °C für 15 bis 25 min durchgeführt wird.

8. Verbindung, welche eine Struktur der Formel (I) aufweist: 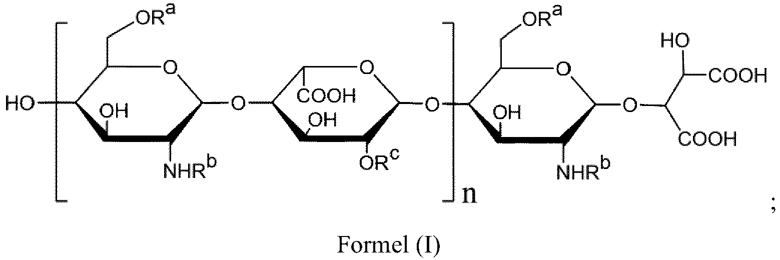 wobei jedes R^{a} unabhängig -SO₃H oder -H ist, jedes R^{b} unabhängig H, -SO₃H oder - C(O)CH₃ ist, jedes R^{c} unabhängig -SO₃H oder -H ist und n 0, 1, 2, 3, 4 oder 5 beträgt.

9. Verbindung nach Anspruch 8, wobei die Verbindung eine der folgenden Strukturen aufweist: 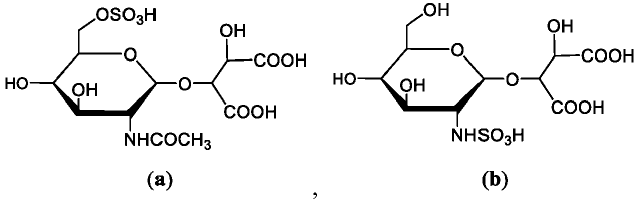 und

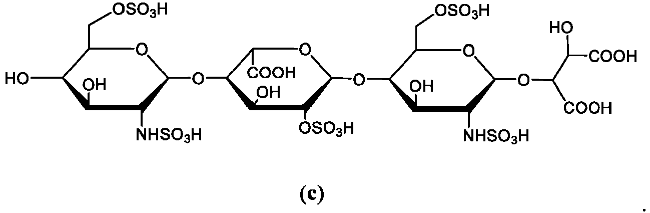

10. Verwendung des Verfahrens zum Detektieren eines carboxylierten Glycosaminoglycan-Derivats nach einem der Ansprüche 1 bis 7 in einer pharmakokinetischen Studie eines carboxylierten Glycosaminoglycan-Derivats.

11. Verwendung des Verfahrens zum Detektieren eines carboxylierten Glycosaminoglycan-Derivats nach einem der Ansprüche 1 bis 6 in einer Qualitätsprüfung einer pharmazeutischen Zubereitung eines carboxylierten Glycosaminoglycan-Derivats.

## Revendications

1. Procédé pour détecter la teneur en un dérivé de glycosaminoglycane carboxylé d'un échantillon, comprenant les étapes suivantes :
(1) hydrolyser un échantillon contenant un dérivé de glycosaminoglycane carboxylé pour obtenir un hydrolysat contenant le composé de la formule (I) : 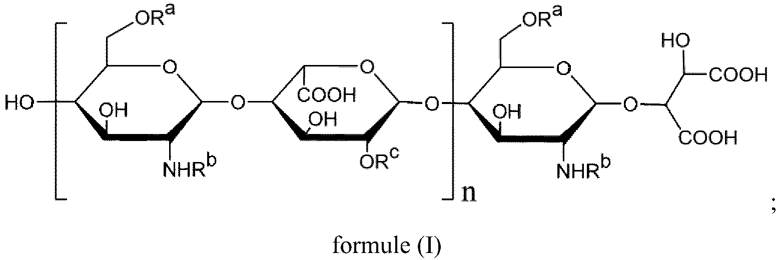 dans laquelle, chaque R^{a} est indépendamment -SO₃H ou -H, chaque R^{b} est indépendamment H, -SO₃H ou -C(O)CH₃, chaque R^{c} est indépendamment -SO₃H ou -H, et n est 0, 1, 2, 3, 4 ou 5 ;
(2) détecter l'hydrolysat obtenu à l'étape (1) par chromatographie liquide couplée à la spectrométrie de masse en tandem ; et
(3) hydrolyser les solutions contenant différentes gradients de concentrations du dérivé de glycosaminoglycane carboxylé comme standard selon le procédé de l'étape (1) ; détecter les zones de pics de signal de masse spectrale du composé de la formule (I) dans les hydrolysats des solutions contenant différentes concentrations du standard selon le procédé de l'étape (2) ; établir une courbe standard entre les teneurs du dérivé de glycosaminoglycane carboxylé standard et les zones de pics de signal de masse spectrale ; et calculer la teneur en dérivé de glycosaminoglycane carboxylé standard de l'échantillon sur la base de la courbe standard et de la zone de pic de signal de masse spectrale du composé de la formule (I) déterminés selon le procédé de l'étape (2) ;
dans lequel, le dérivé de glycosaminoglycane carboxylé est un composé glycosaminoglycane comprenant une unité structurelle de la formule (II) et de façon optionnelle une unité structurelle de la formule (III) : 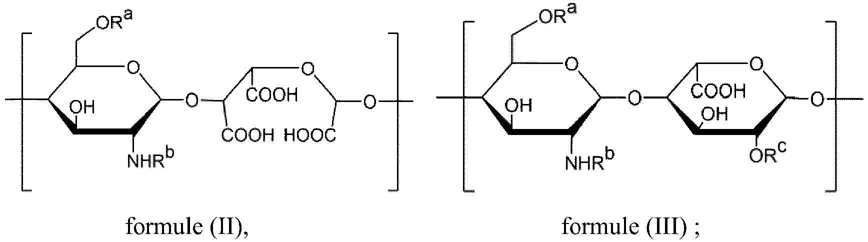
dans lesquelles, chaque R^{a} est indépendamment -SO₃H ou -H, chaque R^{b} est indépendamment H, -SO₃H ou -C(O)CH₃, chaque R^{c} est indépendamment -SO₃H ou -H.

2. Procédé selon la revendication 1, dans lequel
le dérivé de glycosaminoglycane carboxylé est obtenu par réaction d'oxydation en deux étapes, comprenant : (1) oxyder deux groupes alcool adjacents sur l'acide uronique du glycosaminoglycane et effectuer une ouverture de cycle pour former une structure dialdéhyde, et (2) oxyder encore plus la structure dialdéhyde pour obtenir une structure acide dicarboxylique ;
dans lequel le glycosaminoglycane est de l'héparine ou de l'héparane sulfate.

3. Procédé selon la revendication 1, dans lequel le composé de la formule (I) est sélectionné au sein d'un groupe constitué des formules structurelles suivantes : 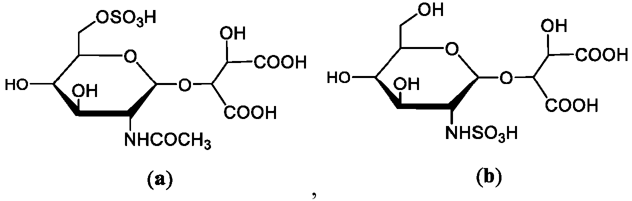 ou

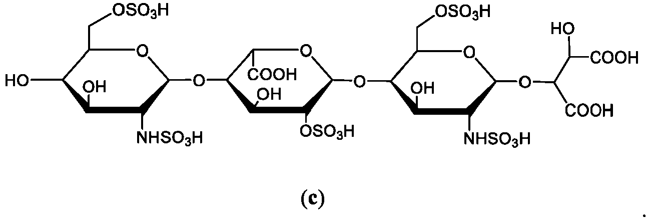

4. Procédé selon la revendication 1, dans lequel le dérivé de glycosaminoglycane carboxylé est doté d'un poids moléculaire moyen en masse de 3000-20000 Da, préférablement de 7000-14000 Da, et plus préférablement de 8000-13500 Da ;
le dérivé de glycosaminoglycane carboxylé est doté d'un degré d'ouverture de cycle de 10-100%, préférablement de 25-80%, et plus préférablement de 25-60%.

5. Procédé selon l'une quelconque des revendications 1 à 4, dans lequel à l'étape (1), le procédé hydrolyse de l'échantillon contenant un dérivé de glycosaminoglycane carboxylé est le chauffage ;
préférablement, le chauffage est réalisé à 70-100°C, préférablement 85-95°C ;
préférablement, le chauffage est réalisé pendant 12-168 h, préférablement 12-120 h.

6. Procédé selon l'une quelconque des revendications 1 à 5, dans lequel la chromatographie en phase liquide est une chromatographie en phase inverse, une chromatographie d'exclusion stérique ou une chromatographie hydrophile ;
préférablement, les phases mobiles de la chromatographie en phase liquide sont la phase mobile A et la phase mobile B ; la phase mobile A est une solution aqueuse d'hexafluoroisopropanol et de pentylamine ; la phase mobile B est une solution aqueuse d'acétonitrile d'hexafluoroisopropanol et de pentylamine ;
préférablement, la phase mobile A est une solution aqueuse contenant 45-55 mM d'hexafluoroisopropanol et 13-17 mM de pentylamine ; la phase mobile B est une solution aqueuse d'acétonitrile contenant 45-55 mM d'hexafluoroisopropanol et 13-17 mM de pentylamine ; la phase mobile B est dotée d'un rapport volumique d'acétonitrile à eau de 70 : 30-80 : 20 ;
préférablement, les phases mobiles de la chromatographie en phase liquide sont la phase mobile A et la phase mobile B, telles que représentées au tableau ci-dessous.
| | |
|---|---|
| Phase mobile A | 50 mM d'hexafluoroisopropanol, 15 mM de pentylamine, H₂O |
| Phase mobile B | 50 mM d'hexafluoroisopropanol, 15 mM de pentylamine, d'acétonitrile/H₂O (75/25, v/v) |

7. Procédé selon l'une quelconque des revendications 1 à 6, dans lequel, lorsque l'échantillon contenant un dérivé de glycosaminoglycane carboxylé est un échantillon biologique, l'hydrolysat obtenu à l'étape (1) nécessite un prétraitement avant détection ; le prétraitement comprend : mélanger l'hydrolysat à une solution d'acide trifluoroacétique et à une solution d'acétonitrile-méthanol, puis placer au repos et centrifuger, collecter le surnageant et sécher, puis re-dissoudre dans l'eau ;
préférablement, l'échantillon biologique comprend du sang et de l'urine ;
préférablement, la solution d'acide trifluoroacétique est ajoutée selon une quantité de 0,5-1,5% d'un volume de l'hydrolysat ;
préférablement, la solution d'acide trifluoroacétique est d'une concentration de 4-6% ;
préférablement, la solution d'acétonitrile-méthanol est ajoutée selon une quantité de 1-5 fois le volume de l'hydrolysat, préférablement 1-3 fois, et plus préférablement 1,5-2,5 fois ;
préférablement, le rapport volumique de l'acétonitrile au méthanol dans la solution d'acétonitrile-méthanol est de 1 : 0,5-1 : 1,5 ;
préférablement, la mise au repos est réalisée de -25 à -15°C pendant 15-25 min.

8. Composé, qui est doté de la structure de la formule (I) : 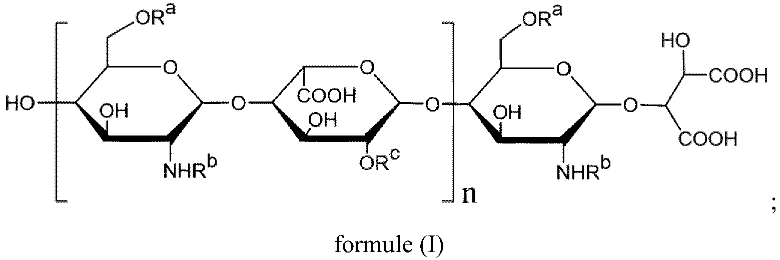 dans laquelle, chaque R^{a} est indépendamment -SO₃H ou -H, chaque R^{b} est indépendamment H, -SO₃H ou -C(O)CH₃, chaque R^{c} est indépendamment -SO₃H ou -H, et n est 0, 1, 2, 3, 4 ou 5.

9. Composé selon la revendication 8, dans lequel le composé est doté de l'une des structures suivantes : 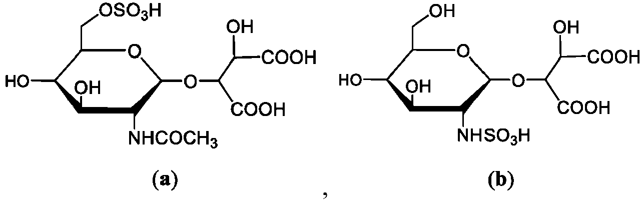 et

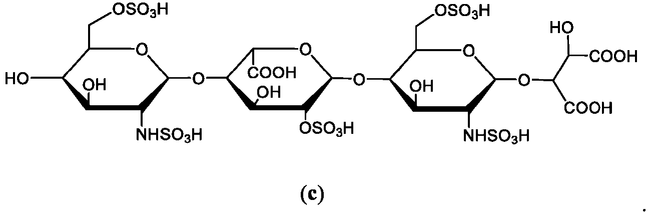

10. Utilisation du procédé pour détecter la teneur en un dérivé de glycosaminoglycane carboxylé d'un échantillon selon l'une quelconque des revendications 1 à 7 dans une étude pharmacocinétique d'un dérivé de glycosaminoglycane carboxylé.

11. Utilisation du procédé pour détecter la teneur en un dérivé de glycosaminoglycane carboxylé d'un échantillon selon l'une quelconque des revendications 1 à 6 dans un essai de qualité d'une préparation pharmaceutique de dérivé de glycosaminoglycane carboxylé.
